(19) Europäisches Patentamt
European Patent Office
Office européen des brevets

(11) **EP 4 202 056 A1**

(12) **EUROPEAN PATENT APPLICATION**
published in accordance with Art. 153(4) EPC

(43) Date of publication:
**28.06.2023 Bulletin 2023/26**

(21) Application number: **21894688.7**

(22) Date of filing: **17.11.2021**

(51) International Patent Classification (IPC):
**C12Q 1/6806** (2018.01)    **C12N 15/09** (2006.01)
**C40B 40/06** (2006.01)

(52) Cooperative Patent Classification (CPC):
**C12N 15/09; C12Q 1/6806; C40B 40/06**

(86) International application number:
**PCT/JP2021/042250**

(87) International publication number:
**WO 2022/107814 (27.05.2022 Gazette 2022/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.11.2020   JP 2020191550**

(71) Applicant: **Xforest Therapeutics Co., Ltd.
Kyoto-shi, Kyoto, 602-0841 (JP)**

(72) Inventors:
• **KOMATSU, Richard Kaoru
Kyoto-shi, Kyoto 602-0841 (JP)**

• **URTEL, Georg Christian
Kyoto-shi, Kyoto 602-0841 (JP)**
• **EDELEVA, Evgeniia
Kyoto-shi, Kyoto 602-0841 (JP)**

(74) Representative: **Schüssler, Andrea
Kanzlei Huber & Schüssler
Truderinger Strasse 246
81825 München (DE)**

Remarks:
The complete document including Reference
Table(s) and the Sequence Listing(s) can be
downloaded from the EPO website

(54) **RNA PROBE FOR MUTATION PROFILING AND USE THEREOF**

(57) Provided is a method for analyzing a higher-order structure of RNA, the method comprising the steps of: (a) preparing one or a plurality of RNA probes, each comprising an RNA to be analyzed attached to a barcode sequence; (b) contacting the RNA probes and an RNA modification reagent; and (c) detecting a position and a frequency of modified nucleotides in a sequence of the RNA probe obtained in step (b), wherein each of the barcode sequences has a structure that has a reduced reactivity with the RNA modification reagent. The method improves the accuracy of detecting mutations, insertions, and deletions, etc., of a base introduced when carrying out mutation profiling using an RNA library.

FIG. 1

Preparation of RNA probe — S10

Modification of RNA probe — S20

Detection of modified bases — S30

Display of detection results — S40

EP 4 202 056 A1

## Description

### Cross References

This application claims a priority based on Japanese Patent Application No. 2020-191550 filed in Japan on November 18, 2020, the entire contents of which are incorporated herein by reference in their entirety. In addition, all the contents described in all patents, patent applications, and documents cited in the present application are incorporated herein by reference in their entirety.

### Technical Field

The present invention relates to an RNA probe for mutational profiling, and more specifically, to an RNA probe comprising an RNA to be analyzed attached to a structured barcode sequence and a method for analyzing the higher-order structure of RNA using the RNA probe.

### Background Art

RNA is a biomolecule that functions as a template for protein synthesis. On the other hand, RNA itself forms a complexed higher-order structure that regulates gene expression, subcellular localization of transcripts, and splicing mechanisms. Many of these functional RNAs are specified by the specific three-dimensionally arrangement of nucleotides defined as respective primary sequences through structure formation. These RNA higher-order structures are formed from combinations of diverse structural motifs, such as STEM, STEM-LOOP, and KISSING-GROUP, MULTI-JUNCTION, KINK-TURN, PSEUDOKNOT, and QUADRUPLEX. The number of types and combinations of these structural motifs are enormous and difficult to predict because they can take on multiple equilibrium states. In response to this, a technique has been developed to determine the higher-order structure of RNA by combining chemical modification reactions to specific bases and sequence data obtained by next-generation sequencing.

For example, mutational profiling (Map), such as the SHAPE-Map method (see Patent Literature 1), which selectively modifies the carbon at position 2 of the sugar of nucleic acids, and the DMS-MaPseq method (see Non-Patent Literature 1), which uses dimethyl sulfate (DMS), are used to estimate the secondary structure of RNA. The distribution of chemical modifications correlates with the secondary structure of the RNA and is recorded as mutations by position-specific reverse transcription termination, substitution, insertion or deletion when sequencing the complementary DNA.

Mutational profiling can be integrated with next-generation sequencing to allow simultaneous analysis of a wider range of RNA types. For example, in the DMS-MaPseq and SHAPE-Map methods, DNA fragments derived from mutated RNAs are mapped against a reference genome on a computer. This operation sorts sequences in conditions where multiple types are mixed, allowing simultaneous counting of structure-specific mutations for RNAs from multiple regions and different molecules. Also, in the PORE-cupine method and the like, a nanopore sequencer can count plurality of mutations in the same molecule by detecting the direct change in potential given by the modified species (see, for example, Non-Patent Literature 2). However, because they use a mapping operation to the reference genome for sequence sorting, they have the disadvantage that it is not clear which genomic location the sequence comes from when similar sequences exist. Examples include gene families and allele-specific RNAs. In addition, the mutagenesis with RNA modification reagents can increase the diversity of similar sequences, thus increasing this effect.

### Citation List

### Non-Patent Literature

[Non-Patent Literature 1] Megan Zubradt et al. DMS-Mapseq for genome-wide or targeted RNA structure probing in vivo. Nat Methods. 14, 75-82 (2017)
[Non-Patent Literature 2] Aw, J.G.A., Lim, S.W., Wang, J.X. et al. Determination of isoform-specific RNA structure with nanopore long reads. Nat Biotechnol (2020). https://doi.org/10.1038/s41587-020-0712-z

### Patent Literature

[Patent Literature 1] JP 6612220 B

**Summary of Invention**

**Technical Problem**

When mutational profiling by chemical modification as described above is performed on an RNA library containing multiple RNAs, there was a problem for RNAs that differ by only one or several nucleotides. The problem is that it is difficult to distinguish by the sequence alignment of cDNA between mutations caused by chemical modifications and the natural diversity of sequences that could be included in the library.

The present invention is intended to solve the problem that arises upon mutational profiling using RNA libraries, which problem is to improve the detection accuracy of introduced mutations, insertions, deletions and the like of nucleotides without affecting the higher-order structure of the RNA to be analyzed.

**Solution to Problem**

The present invention was made to solve such problems, that is, when performing mutational profiling, a barcode sequence is added to each RNA in the RNA library. Each barcode sequence is unique in the RNA library and has a structure that inhibits the reaction with chemical modifiers.

That is, in the first aspect of the present invention, there is provided a method for analyzing the higher-order structure of RNA. The method comprises the steps of (a) preparing one or a plurality of RNA probes, each comprising an RNA to be analyzed attached to a barcode sequence, (b) contacting the RNA probes and an RNA modification reagent, (c) detecting a position and a frequency of modified nucleotides in a sequence of the RNA probe obtained in step (b). Each of the barcode sequence is characterized by having a structure that has a reduced reactivity with the RNA modification reagent. The above detection process (c) preferably includes the following steps.

(c1) synthesizing complementary DNAs using a mixture of the RNA probes obtained in step (b) as a template by a reverse transcriptase, (c2) determining nucleotide sequences of the complementary DNAs and aligning the nucleotide sequences comprising the barcode sequence, and (c3) detecting a position and a frequency of mutations that occurred in the aligned nucleotide sequences.

In another aspect of the present invention, there is provided an RNA probe containing RNA to be analyzed attached to a barcode sequence that forms a structure containing a plurality of base pairs, and an RNA probe library including a plurality of the RNA probe. In a further embodiment, a group of RNA probe libraries comprising two or more replicates of this RNA probe library is provided. All replicated RNA probes additionally contain a second barcode sequence, which is identical within one library but distinguishable from other libraries.

**Effect of the Invention**

According to the method of the present invention, when mutational profiling is performed using RNA libraries, the detection accuracy of introduced mutations, insertions, and deletions of nucleotides can be improved without affecting the higher-order structure of the RNA to be analyzed.

**Brief Description of the Drawings**

FIG. 1 is a flow chart illustrating a method for analyzing the higher-order structure of RNA according to an embodiment.

FIG. 2 is a flow chart illustrating a method for analyzing the higher-order structure of RNA in another embodiment.

FIG. 3 is a schematic diagram showing (a) the barcode sequence used for the preparation of the first library and (b) the outline of the library structure.

FIG. 4 is a schematic diagram showing an outline of a library structure produced by using 37 kinds of the first barcode sequences and 4 kinds of the second barcode sequences (batch barcodes).

FIG. 5 shows the sequences of two samples (ID1 and ID32) synthesized as individual strands among RNA probes contained in the first library.

FIG. 6 is a schematic diagram showing the flow of mutational profiling operations performed with the second library.

FIG. 7 shows the absolute value of delta mutation rate of all nucleotides in the barcode of samples chemically modified with NAI or DMS. The results were shown separately in the structured barcode (ID1-28) and the unstructured barcode (ID29-37) in the RNA probe in the first library.

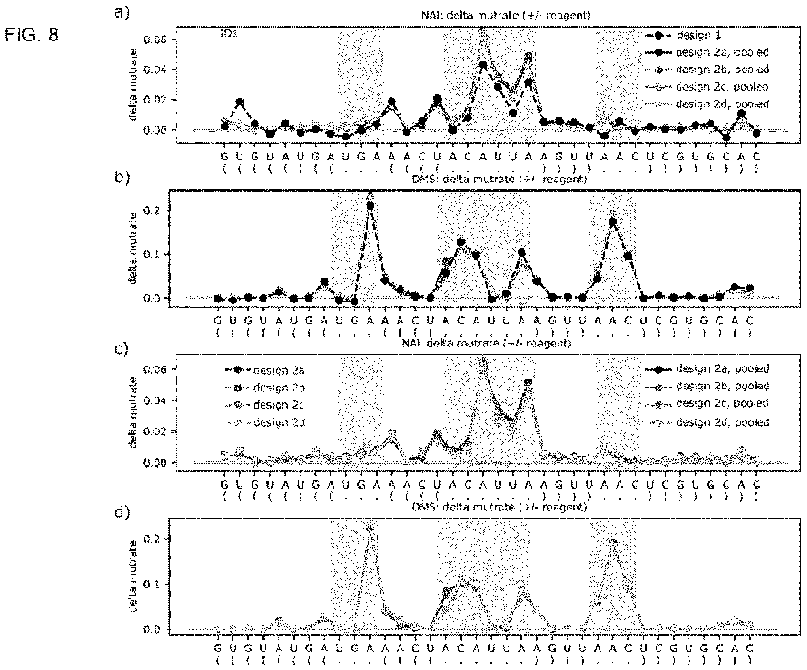

FIG. 8 shows the results of delta mutation rates for each nucleotide when each library is chemically modified with NAI or DMS. The x-axis shows the sequence of the target RNA of ID1 and the predicted structure in dot-bracket notation. (a) is a result when the first library and four kinds of second libraries were treated with NAI. (b) is a result when the first library and four kinds of second libraries were treated with DMS. (c) is a result when the second library was treated with NAI, either individually or pooled together. (d) is a result when the second library was treated with DMS, either individually or pooled together.

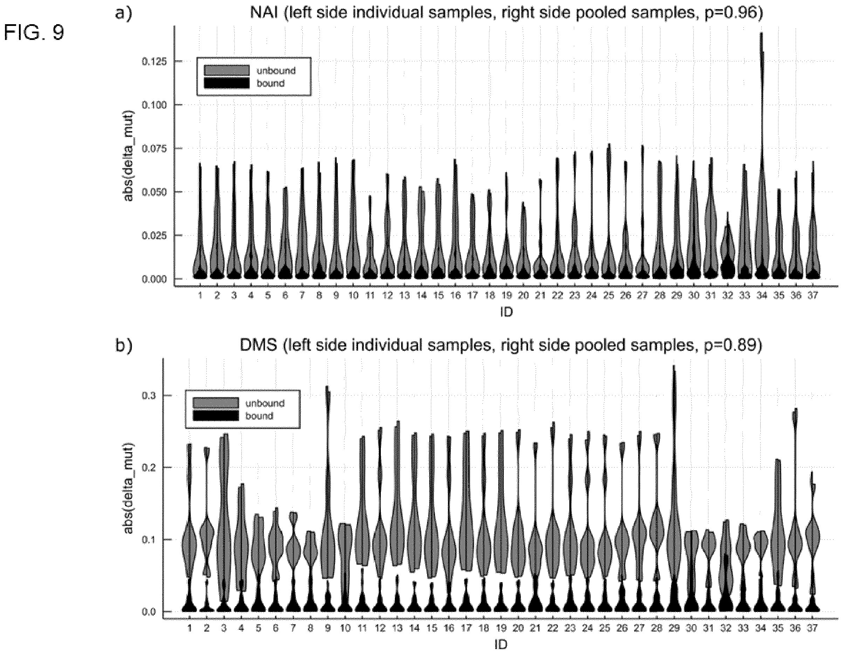

FIG. 9 are violin plots showing kernel density distribution of delta mutation rates of nucleotides predicted to be bound (black portions) and unbound (grey portions) for delta mutation rates of each ID when the second library is chemically modified with NAI or DMS, either individually or pooled together. Samples of (a) are treated with NAI, and samples of (b) are treated with DMS. The left side of each "violin" is the distribution of four individual samples taken together, and the right side is the distribution of pooled samples. For samples treated with DMS, the numerical calculations considered only C and A mutations.

FIG. 10 is a graph in which mutation profiling is performed without a modifier using an RNA probe library group to which a structured batch barcode is added, and the number of reads obtained in the next generation sequence of the RNA probe library to which ID1 is added is plotted for each ID when all reads obtained in the next generation sequence of the RNA probe library to which ID1 to 96 barcodes are added are mapped for the file of the RNA probe library group to which IDs are added.

FIG. 11 is a graph in which mutation profiling by DMS is performed using an RNA probe library group to which a structured batch barcode is added, and the number of reads obtained in the next generation sequence of the RNA probe library to which ID2 is added is plotted for each ID when all reads obtained in the next generation sequence of the RNA probe library group to which barcodes of ID1 to 96 are added are mapped for the file of the RNA probe library group to which the barcode is added.

FIG. 12 shows the results of mutation profiling using the RNA probe library group to which the structured batch barcode is added without a modifier, and plotting the percentage determined to be the correct ID for each RNA.

FIG. 13 shows the results of performing mutation profiling by DMS using an RNA probe library group to which a structured batch barcode is added, and plotting the percentage determined to be a correct ID for each RNA.

FIG. 14 is a graph in which the number of reads obtained from an RNA probe library to which ID7 is assigned is plotted for each ID when mutation profiling is performed using a structured batch barcode, and then next generation sequencing is performed in combination with a plurality of indexes, and all reads obtained from the RNA probe library to which ID1 to 96 barcodes are assigned are mapped for the group of RNA probe libraries to which ID1 to 96 barcodes are assigned.

FIG. 15 is a graph plotting the number of reads of the structured batch barcode ID mapped to the index ID as a result of next generation sequencing performed by assigning a corresponding index to the structured batch barcode with one-to-one correspondence.

FIG. 16 is a graph plotting the number of types (RNA IDs) of RNAs erroneously determined in the RNA probe library to which each structured batch barcode ID is assigned in FIG. 15.

FIG. 17 is a result of performing next generation sequencing by assigning a corresponding index to a structured batch barcode with one-to-one correspondence, and plotting accuracy in determining an ID of the structured batch barcode for each index.

FIG. 18 is a diagram showing examples (ID12 and ID28) of structured batch barcode sequences used in Example 4.

**Description of Embodiments**

Next, embodiments of the present invention will be described with reference to the drawings. Note that each embodiment described below does not limit the invention according to the claims, and all of the elements described in each embodiment and combinations thereof are not necessarily essential to the solution of the present invention.

(Definition)

As used herein, "RNA to be analyzed" or "RNA of interest" means, interchangeably, an RNA molecule having a sequence that may interact with a low-molecular-weight (small molecule) compound or protein *in vivo*. The RNA to be analyzed may be a biological sample obtained by extraction from a living organism, or it may be artificially synthesized RNA. When the RNA to be analyzed is artificially synthesized, it should preferably contain a motif region, which is a functional structural unit of RNA extracted based on RNA sequence information. The term "motif region" means a functional structural unit for RNA to interact with a target substance. The components of this RNA motif, such as stem-loop and pseudoknot, are called structural motifs, and the combination of these structural motifs forms the higher-order structure of RNA. The motif region comprised in the RNA probe of the present invention may consist of a single stem-loop structure (hairpin loop structure) or may contain a plurality of stem-loop structures (multi-branched loop structure). It may also contain one or more kink-turns, pseudoknot, guanine quadruplex (G-quadruplex), and the like. Structural motifs can be composed not only by Watson-Crick base pairs but also by Hoogsteen base pairs.

"RNA probe" refers to a nucleic acid molecule containing RNA to be analyzed, preferably a nucleic acid molecule consisting of RNA, to which a primer binding site for amplification or a barcode sequence is added. The term "library" refers to a set of a plurality of (two or more) different types of molecules (e.g., a plurality of different DNA molecules or a plurality of different RNA molecules, etc.). In this embodiment, the term "library" can preferably include more than 10 different RNA molecules, more preferably more than $10^2$, $10^3$, or $10^4$, and even more preferably more than $10^6$ different RNA molecules, to allow the analysis to be performed using a large number of RNA probes as needed.

The term "higher-order structure of RNA" refers mainly to the partial double-strand formation based on intramolecular base pairing (also called stem structure), the single-strand structure or cyclic single-strand structure without base pairing (called loop structure), or a combination thereof in solution. Such structures are in a specific equilibrium state depending on the solution conditions (temperature, salt concentration, etc.) and fluctuate with the movement of the RNA molecule. The term "stem structure" refers to a double helical structure formed by any nucleic acid sequence contained in an RNA and a sequence complementary to the nucleic acid sequence. As used herein, the term "complementary" means that two nucleic acid sequences can hybridize with each other. The two nucleic acid sequences constituting the stem structure may be complementary at least 50%, 55%, 60%, 65%, 70%, 75%, 80%, 85%, 90%, 95%, 99% or 100% in sequence to each other, because the two sequences may hybridize with each other.

The term "barcode sequence" is a tag having a unique sequence that is added to each nucleic acid molecule, or to each type of nucleic acid molecule. It is also referred to as an "index" or "Unique Molecular Identifier (UMI)". UMIs are typically intended to improve quantitation by reducing amplification bias by assigning a random sequence to each molecule in solution. If barcode sequences with a unique sequence for each kind of RNA are added to a plurality of RNAs to be analyzed, after simultaneous modification and amplification of a plurality of RNAs, each RNA can be identified and analyzed based on the kind of added barcode. In addition, by identifying each experimental series based on different barcodes for each reaction solution and reaction condition, a plurality of experimental data can be obtained separately from the same next-generation sequence data, enabling efficient data analysis.

Barcode sequences can be provided, for example, as a group of nucleic acids with random base sequences. The barcode sequences may be synthesized randomly (so that the sequence is diverse, and the sequence information does not need to be recognized), since the number of sequence variations is important. Alternatively, the barcode sequence may be a group of nucleic acids with known sequences designed to provide sufficient diversity.

(Methods for analyzing the higher-order structure of RNA)

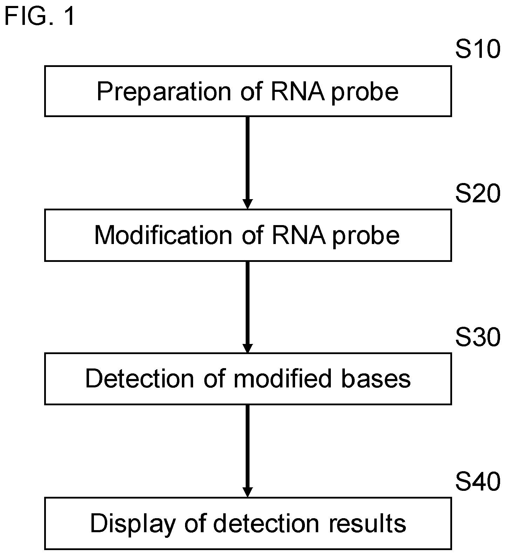

FIG. 1 is a flow chart of a method for analyzing the higher-order structure of RNA in one embodiment of the present invention. This method includes the steps of preparing one or a plurality of RNA probes, each comprising an RNA to be analyzed attached to a barcode sequence (S10), contacting the RNA probes and an RNA modification reagent (S20), detecting a position and a frequency of modified nucleotides in a sequence of the RNA probe obtained in step S20 (S30), and if necessary, displaying the detection results (S40). Here, the barcode sequence is characterized by having a structure that has a reduced reactivity with the RNA modification reagent.

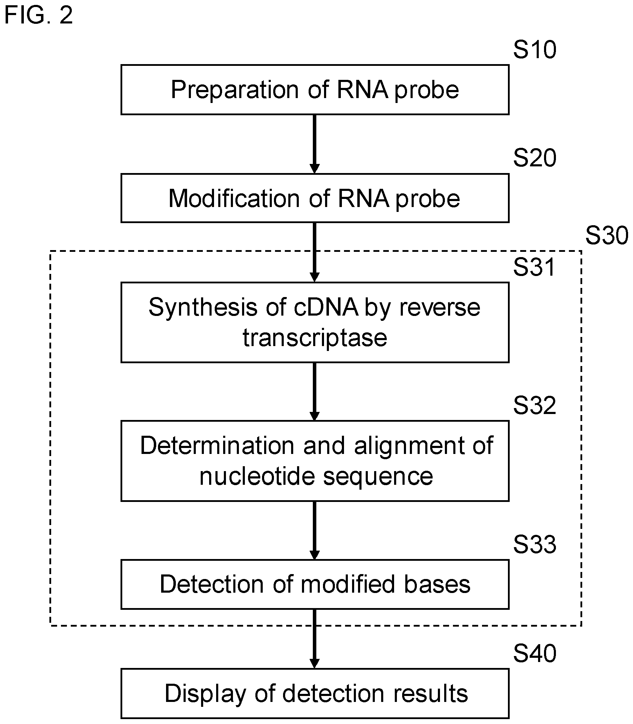

In another embodiment of the method for analyzing the higher-order structure of RNA, as shown in FIG. 2, the detection step (S30) preferably comprises the steps of synthesizing complementary DNA by reverse transcriptase using the mixture of RNA probes obtained in step S20 as a template (S31), determining the nucleotide sequence of the complementary DNA and aligning the nucleotide sequence containing the barcode sequence (S32), and detecting the position and frequency of a mutation generated in the aligned nucleotide sequence (S33). Hereinafter, each of these steps will be described in detail.

<Preparation of RNA probe (S10)>

The RNA to be analyzed preferably contains a motif region for its function *in vivo.* This motif region may comprise a single stem-loop structure (hairpin loop structure) or may comprise multiple stem-loop structures (multi-branched loop structures). In the present embodiment, it is preferable to extract a motif region based on the stem structure (see, for example, WO2018/003809 specification). This allows the preparation of RNA probes that reflect the functional structural units that exist in RNA without fragmenting the motif regions. The motif region may have any sequence length as long as its function is maintained, and may be, for example, 1000 bases or less, 900 bases or less, 800 bases or less, 700 bases or less, 600 bases or less, 500 bases or less, 400 bases or less, 300 bases or less, 200 bases or less, 150 bases or less, 100 bases or less, or 50 bases or less.

In the above method for preparing an RNA probe containing the RNA to be analyzed, the stem structure in the RNA can be obtained and recognized, for example, by using an RNA secondary structure prediction software such as CentroidFold (Hamada, M. et al., Bioinformatics, Vol. 25, pp 465-473, 2009) or IPknot (Sato, K. et al., Methods Biochem. Anal., Vol. 27, pp. i85-i93, 2011). In addition, it is possible to use any RNA sequence information, for example, one downloaded from an RNA sequence data base such as UTRdb (Grillo, G. et al., Nucl. Acids Res., Vol. 38, D75-D80, 2010), IRESite (Mokrejs, M. et al., Nucl. Acids Res., Vol. 38, D131-D136, 2010), GenBank (Benson, D. et al., Nucl. Acids Res., Vol. 41, D36-D42, 2013) or RNAcentral (RNAcentral Consortium, Nucl. Acids Res., Vol. 43, D123-D129, 2015). RNA sequence information may be also obtained from databases containing not only RNA sequence information but also RNA structure information. For example, the RNA sequence information downloaded from Rfam (Nawrocki, E. P. et al., Nucl. Acids Res., Vol. 43, D130-D137, 2015), Structure Surfer (Berkowitz, N. D. et al., BMC Bioinformatics, Vol. 17, p. 215, 2016) or the like can be used. Three-dimensional RNA structure data determined by various methods may also be used. For example, the data can be downloaded from Protein Data bank (https://www.rcsb.org/). The self-designed RNA higher-order structure can also be used, for example, the data designed by the software such as RNAinverse.

In this step, barcode sequences added to the RNA to be analyzed are structured. The term "structured" means that the RNA folds in solution to form a secondary or tertiary structure or remains in a primary structure (sequence), thereby reaction with RNA modification reagents is inhibited. For example, when the RNA modification reagent is a compound that selectively modifies unbound (unconstrained) nucleotides, such as single-stranded regions in RNA probes, the barcode sequences can be designed to form a structure containing a plurality of base pairs that are not susceptible to such modification. A plurality of base pairs means that two or more consecutive or separated bases form hydrogen bonds with other bases in each of the barcode sequences and can be either Watson-Crick or Hoogsteen base pairs. G-U wobble base pairing, which has the same level of thermodynamic stability as Watson-Crick base pairing, is also acceptable. In the same environment or condition as in *vivo,* two or more base pairs can form a stable structure at least temporarily, but three or more base pairs are preferable, four or more base pairs are more preferable, and five or more base pairs are still more preferable in order to form a more stable structure. The upper limit of the number of base pairs is not particularly limited, but since a sufficiently stable structure can be obtained if about 10 base pairs are present, from the viewpoint of cost, 30 or less base pairs are preferable, 20 or less base pairs are more preferable, and 15 or less base pairs are still more preferable. On the other hand, when the RNA modification reagent selectively modifies the bound (constrained) nucleotides to form double-stranded nucleotides in the RNA probe, the barcode sequence should be designed to maintain a sequence that does not form base pairs, i.e., a single-stranded structure. Furthermore, the barcode sequence with this structure should be computationally optimized so that the sequence does not affect the RNA to be analyzed. This is to avoid the problem that the barcode sequence itself may form a structure that is far from the original RNA structure due to intramolecular interactions with the RNA to be analyzed or may affect the stability of the structure. Computational sequence optimization can be performed using known programs such as the ViennaRNA package or other known programs.

<Modification of RNA Probe (S20)>

The RNA modification reaction in this step (S20) is carried out by bringing the RNA probe prepared in the previous step (S10) into contact with the desired RNA modification reagent. In one embodiment, the RNA modification reagent is a compound that selectively modifies unbound nucleotides, such as single-stranded regions in RNA probes. Such compounds are typically known as SHAPE reagents and comprise but not limited to isatoic anhydride derivatives that react to ribose-2'-hydroxy group, such as 1-methyl-7-nitroisatoic anhydride (1M7), 1-methyl-6-nitroisatoic anhydride (1M6), NMIA (N-methylisatoic anhydride ) and 2-methylnicotinic acid imidazolide (NAI). In addition to the SHAPE reagent, dimethyl sulfate (DMS) can be used as an RNA modification reagent to form adducts at N1 position of adenosine, N3 position of cytosine, and N3 position of uridine, and N1 position of guanosine. As an example, NAI generally reacts with all four nucleotides and DMS reacts only with adenine and cytosine. On the other hand, DMS can also react with guanine and uridine under basic-biased pH conditions (e.g., pH 8.0).

In other embodiments, the RNA modification reagent may be one that selectively modifies the bound nucleotides such that they form double strands in RNA probes. This RNA modification reagent includes but not limited to RNASEV1, and DICER of the RNASEIII family, both of which are enzymes that degrade double-stranded RNAs, or a fusion protein of a double-stranded binding protein and an RNA modifying protein.

A solvent solution containing such an RNA modification reagent is added to the solution containing the RNA probe, and the RNA probe and the RNA modification reagent are brought into contact with each other. The solution may be a biological solution containing different concentrations and amounts of proteins, cells, viruses, lipids, monosaccharides and polysaccharides, amino acids, nucleotides, DNA, and various salts and metabolites. The solution may also contain different concentrations and amounts of low molecular weight or medium molecular weight drug. The composition may also contain various surfactants, polymers, and osmolyte. The concentration of the RNA modification reagent can be adjusted to achieve the desired degree of modification to the RNA.

Furthermore, RNA to be analyzed can be modified in the presence of proteins, or other low molecular weight and high molecule weight biological ligands. If the reactivity of the RNA modification reagent is pH dependent, the pH may be maintained in the range of, for example, but not limited to, 7.5 to 9.0. The functional range that differentiates maximum from minimum reactivity nucleotides typically ranges from 20 to 50-fold. The RNA can be replaced by any procedure that folds into the desired conformation at the desired pH (e.g., about pH 8). The RNA can be heated first and then cooled in a steep, low ionic strength buffer to eliminate multimeric forms. The folding solution can then be added to prepare for the RNA to achieve the correct conformation and search for structure sensitive RNA modification reagents. In some embodiments, the RNA is not naturally folded prior to modification. Modifications may be made while the RNA is denatured by thermal and/or low salt conditions.

<Detection of modified bases (S30)>

This step detects the position and frequency of modified bases in the RNA probe sequence obtained in the above modification process (S20). The method for reading the modified bases in the RNA sequence is not limited, and can be, for example, the pull-down method using an antibody specific for the modified base, and the nanopotential reading method that measures the electric potential of the RNA. This direct RNA nanopore sequencing method is a technique for detecting RNA modification sites at the single molecule level. Currently, the direct RNA sequencing platform developed and marketed by Oxford Nanopore Technologies reads RNA bound to motor protein via membrane-suspended biological nanopores. As the RNA passes through the pore under voltage bias, a short sequence (5 nucleotides) depending on the chemical identity (i.e., sequence), a change in ion current of picoamperes is observed (see Garalde, D. R., et al. (2018) Highly parallel direct RNA sequencing on an array of nanopores. Nat. Methods, and Workman, R.E., et al. (2019) Nanopore native RNA sequencing of a human poly(A) transcriptome. Nat. Methods, 16, 1297-1305). It has been reported that nucleotides modified by 1-acetylimidazole (AcIm), one of the SHAPE reagents, can be detected by this method (William Stephenson et al., Direct detection of RNA modifications and structure using single molecule nanopore BioRxiv doi: https://doi.org/10.1101/2020.05.31.126763, Posted June 01, 2020) .

In a preferred embodiment, the step of detecting modified bases (S30) is mutational profiling comprising the conversion of RNA to complementary DNA (cDNA), as shown in FIG. 2. In this embodiment, cDNA is first synthesized by reverse transcriptase or another polymerase using the mixture of RNA probes obtained in step S20 as a template (S31). Reverse transcriptase is an enzyme that synthesizes cDNA from RNA, and includes, but is not limited to, a thermostable enzyme such as mouse or avian reverse transcriptase. Alternatively, the enzyme may be a reverse transcriptase TGIRT (Thermostable Group II intron reverse transcriptase) present in retrotransposons such as prokaryotes and fungi. TGIRT-III from InGex has superior thermal stability, processability, and accuracy compared to conventional retroviral reverse transcriptase. In addition, a property of inducing a mutation at a site modified by DMS at the time of reverse transcription is known (DMS-MaPseq method).

These enzymes include methods of detecting chemical modifications in RNA by skipping nucleotides comprising adducts and incorporating incorrect (non-complementary) nucleotides at sites of chemical modification. As used herein, "incorrect" with respect to nucleotide incorporation refers to incorporation of non-complementary nucleotides (nucleotides that are contrary to Watson-Crick rules) into nucleotides present in the original sequence. This involves a small number of deletions within the sequence.

Subsequently, the nucleotide sequence of the cDNA is determined, and the nucleotide sequence including the barcode sequence is aligned (S32). By using libraries derived from a mixture of a variety of RNA probes, cDNA can efficiently detect chemical modifications in nucleic acids such as RNA using massively parallel sequencing (MPS). As an example, in a next generation sequencer manufactured by Illumina, the 5' end is fixed on a flow cell via adapters at both ends of a DNA fragment of tens to hundreds of millions. Next, the adapter on the 5' end side previously fixed on the flow cell and the adapter sequence on the 3' end side of the DNA fragment are annealed to form a bridge-shaped DNA fragment. By performing a nucleic acid amplification reaction using a DNA polymerase in this state, a large number of single-strand DNA fragments can be locally amplified and fixed. Then, in the next generation sequencer, sequencing is performed using the obtained single-strand DNA as a template, and thus, it is possible to obtain huge sequence information of about 3 Tb in one analysis currently in 2020. These techniques for fast and parallel reading of nucleic acids are also referred to as "next-generation sequencing (NGS)", "ultra-parallel sequencing", "ultra-high throughput gene sequencing (Ultra-High-Throughput sequencing)", "large-scale parallel sequencing", or the like.

In one embodiment, the sequence data (reads) obtained by the next generation sequencer are aligned in a form including a barcode sequence. This is because, by aligning sequence data for each individual barcode sequence, samples containing a large number of types of RNA probes can be sequenced simultaneously. Even when the RNA to be analyzed contains similar sequences, for example, gene families, single nucleotide polymorphisms, and the like, it is possible to discriminate and analyze them.

Alternatively, after all cDNAs are aligned together, the alignment may be evaluated by adding the barcode mutation information to the alignment with low reliability. In any of the methods, the accuracy of the sequence information can be improved by aligning the sequence of RNA to be analyzed with the barcode sequence.

The position and frequency of the mutation generated are detected based on the nucleotide sequence aligned in this manner (S33). The mutation rate at a given nucleotide is simply the number of mutations (mismatch, deletion and insertion) divided by the number of reads at that location. The raw reactivity calculation data for each nucleotide can be normalized using various criteria. Data quality control is possible by taking into account the reading depth of the sequence and the standard error.

<Display of Detection Results (S40)>

The position and frequency of the mutation detected in the above step can be illustrated by methods known to those skilled in the art such as a histogram of mutation frequencies, a sequence depth, and a reactivity profile. Alignment software such as BWA and STAR can be used for analysis of mutation positions and frequencies. These data are converted into numerical values and vectors as mutation counts, and various calculations can be performed. Mutations that show statistically significant reactivity can be annotated.

These analyses in this step can be performed using a computer program product stored on a computer readable medium. Exemplary computer readable media suitable for implementing the present invention include chip memory devices, disk storage devices, programmable logic devices, and application specific integrated circuits. Further, the computer program product implementing the present steps can be installed on a single device or computing platform or distributed among multiple devices or computing platforms. Therefore, the RNA conformation obtained by the method of the present embodiment can be displayed on a display connected to a computer.

(Action and Effect)

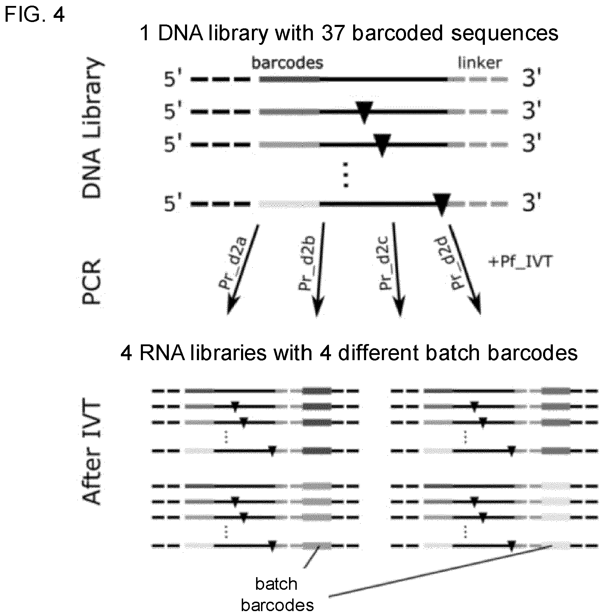

The structured barcode disclosed in the present embodiment has several advantageous actions and effects. One is that the barcode sequence is less likely to be modified in the reaction with RNA modification reagents and can be correctly identified as a barcode. Also, the barcode portion is less likely to interact with RNA to be analyzed or other RNA molecules. This allows the structured barcode sequences not only to be distinguished from similar sequences in a library, but also to distinguish between different batches of the same library. For example, FIG.4 represents a method of preparing a group of libraries using 37 different first barcode sequences and four different second barcode sequences. By amplifying the first library of 37 different DNAs with four different primers, second barcode sequences are added that are the same sequence in one library but have different sequences in different batches of libraries. By using these *in vitro* transcription reactions, a group of RNA libraries comprising two types of different barcode sequences can be produced.

(RNA probe and RNA probe library)

In another embodiment of the present invention, an RNA probe including a structured barcode sequence and an RNA probe library including a plurality of the RNA probes are provided. In one embodiment, the structured barcode sequence is a barcode sequence that forms a structure comprising a plurality of base pairs. The barcode sequence of the present embodiment includes, for example, a complementary double-stranded structure, a triple-stranded structure, or a quadruplex structure, and specifically, a stem-loop structure, a pseudoknot structure, or the like can be exemplified. The stem region forms a complementary duplex but may contain wobble base pairs (G-U, I-U, I-A, and I-C) having the same degree of thermodynamic stability as Watson-Crick base pairs to increase sequence diversity. "I" represents inosine, whose base, hypoxanthine, is capable of base pairing with uracil, adenine, and cytosine. Uracil can pair with two different bases, guanine and adenine.

In other embodiments, the structure comprising a plurality of base pairs is a stem-loop structure with one or more bulges and/or internal loop structures at the stem region. This can increase the diversity and variety of higher-order structures that a structured barcode can take. In addition, bases that serve as negative and positive controls for structure-specific mutations can be loaded at the same time. The structured barcodes can also serve as controls for single-stranded RNA modifiers that modify the terminal loop but not the bulge or inner loop.

In some embodiments, the structure comprising a plurality of base pairs is an RNA structure or a variant thereof that is registered in the protein data bank (PDB). This allows for barcoding of RNA higher-order structures that are not Watson-Crick type. For example, this can be useful as a structured barcode for modification reagents to RNA higher-order structures that are not formed by Watson-Crick base pairs only.

The position of the structured barcode sequence in the RNA probe of the present embodiment is not particularly limited and can be placed at any position. For example, it can be at the 5' end or 3' end of the RNA to be analyzed. Alternatively, one strand of the barcode sequence forming the complementary strand may be positioned at the 5' end of the RNA to be analyzed and the other strand at the 3' end, forming a double strand so that they sandwich the RNA to be analyzed. The number of structured barcode sequences is also not limited, and there may be multiple structured barcodes of the same or different sequences.

The RNA probe of the present embodiment includes an RNA motif including at least one structural motif as the RNA to be analyzed. The motif region may be extracted from any RNA sequence information. Alternatively, a motif region included in the RNA probe of the present invention may be selected from any RNA secondary structure data already identified by RNA structure studies.

Furthermore, the RNA probe may be labeled with a fluorescent dye (e.g., FITC, PE, Cy3, Cy5, etc.), a radioisotope, digoxigenin (DIG), biotin, etc., for detection. Labeling can be performed by incorporating a pre-labeled nucleic acid at the time of probe synthesis, and for example, an artificial nucleic acid labeled on the 5' side can be incorporated. In addition, an artificial nucleic acid labeled over the entire length of the RNA can be incorporated. The 3' side can be labeled with an artificial nucleic acid labeled with, for example, T4 RNA ligase 1. The labeling may be performed in multiple steps by click reaction or the like. For example, DBCO-biotin and DBCO-Cy3 are reacted with an RNA to which pCp-N3 is added to the 3' end using T4 RNA ligase 1, whereby a fluorescent dye or biotin can be incorporated into the RNA. The proportion of these labels may be 10, 20, 30, 40, 50, 60, 70, 80, 90, 99, 100%.

The RNA probe of the present embodiment can be synthesized by any known genetic engineering method. Preferably, the RNA probe can be produced by transcription from a template DNA which has been synthesized by outsourcing to a synthesis outsourcee. For transcription of RNA from DNA, the DNA containing the sequence of the RNA probe may comprise a promoter sequence. Examples of the preferable promoter sequence include, but are not particularly limited to, a T7 promoter sequence. When the T7 promoter sequence is used, for example, RNA can be transcribed from DNA having a desired RNA probe sequence using MEGAshortscript™ T7 Transcription Kit provided by Life Technologies. In the present invention, RNA can be not only adenine, guanine, cytosine, and uracil, but also modified RNA. Modified RNAs include, for example, pseudouridine, 5-methylcytosine, 5-methyluridine, 2'-O-methyluridine, 2-thiouridine, and N6-methyladenosine are examples.

In one embodiment, an RNA probe library, containing a plurality of RNA probes each containing a different sequence of RNA to be analyzed is provided. In the present embodiment, it is preferable to prepare many kinds of RNA probes at the same time and to use an oligonucleotide library synthesis (Oligonucleotide Library Synthesis) technique that efficiently contains the templates for the RNA probes. This is accomplished by synthesizing one base at a time using an inkjet technique that prints individual bases at defined positions on a slide to elongate template DNA of a specified length. The synthesized oligos are then cut from the slide, pooled, dried and stored in one tube. The oligo library can then be reconstituted and amplified before preparing the RNA probe library by *in vitro* transcription reactions. Oligonucleotide Library Synthesis, which is not specifically limited in this invention, can be prepared by outsourcing to Agilent Technologies or Twist Biosciences, Inc.

In still another embodiment, a group of RNA probe libraries consisting of two or more replicates can be prepared by amplifying an RNA probe library of this embodiment containing a plurality of RNA probes with multiple primers each containing a second barcode sequence. All replicated RNA probes contain first and second barcode sequences, in which the second barcode sequences are all identical within one library but distinguishable from other libraries. According to the examples described later, even when a plurality of RNA probe libraries are mixed and mutational profiling is performed, the same results as those obtained when each RNA probe library is used are obtained, and thus it is considered that each mutational profiling can be identified using the second barcode sequence after performing different mutational profiling using each RNA probe library and then performing next generation sequencing after mixing them. Therefore, by adding the second barcode sequence, even when mutational profiling is performed under different reaction conditions, a next generation sequencing library can be prepared under the same conditions, and the efficiency of the mutational profiling process can be particularly improved. It is also possible to prepare an RNA probe library group containing three or more barcode sequences by further adding different barcode sequences to the RNA probe library group containing the first and second barcode sequences.

The RNA library of this embodiment can be used as a kit for analysis of chemical modifications of RNA and/or RNA structure analysis. The method of using such a kit includes the method of analyzing the higher-order structure of RNA in accordance with the present invention. The following examples are provided to explain the invention in more detail, but the invention is not restricted in any way to these examples.

[Example 1]

Materials and Methods

(Design of barcode sequences)

For the barcodes, different stem and loop lengths were used. Stems of length 6, 7 and 8 base pairs (bp) were generated randomly, allowing canonical base pairs and GU wobbles. For each stem length, three different loop lengths were used. Either random 3 or 5 nucleotide long sequences or one of four tetraloops (UUCG, GAGA, GCUU, GUAA) or one of sequences of 3 or 5 bases in length (UCG, AGA, CUU, UAA, UUACG, GAAGA, GCUAU, AGUAA) was chosen for each barcode. Using the ViennaRNA package, the barcodes were controlled for correct folding. As control, unstructured barcodes were generated with lengths of 10, 15 and 21 nucleotides in length.

(RNA sequence of interest)

To demonstrate the usefulness of the structured barcode, the following sequence was used as the RNA of interest:
5'-GUGUAUGAUGAAACUACAUUAAGUUAACCUCGUGCAC-3' (SEQ ID NO:1). From this initial sequence, 36 point mutants were derived by choosing 12 unpaired positions and making all three possible point mutants. This gives 37 sequences in total. The difference between any pair of this sequences is either one or two-point mutations.

(Design of the first library)

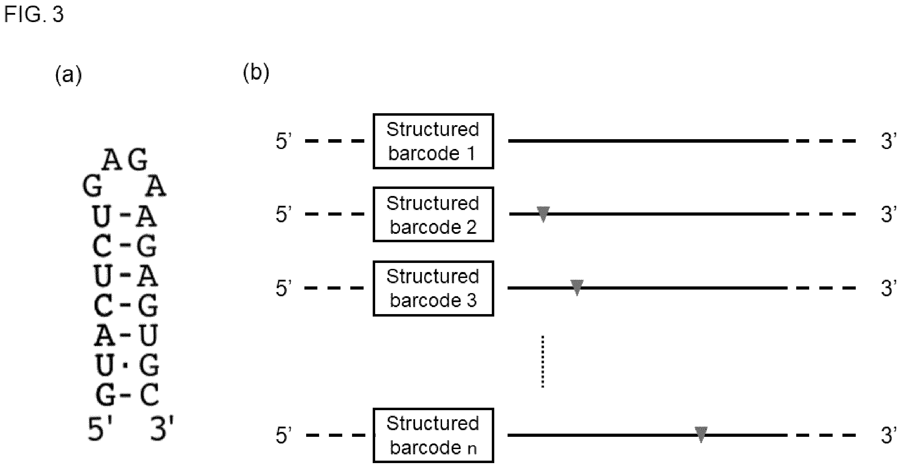

An outline of the barcode sequence used for the first library and the library structure are shown in FIG. 3. FIG. 3(a) is a barcode sequence of one RNA probe (ID1) and is composed of a seven bp stem and a four nucleotide loop. The first library sequences have four parts from 5' to 3':

i) A 5'-cassette required for generating the RNA library via *in-vitro* transcription (IVT) and for the library preparation for sequencing. (broken line on the 5' side in FIG. 3(b));
ii) Different barcode sequences for each individual sequence (ID1 to ID28 including the structured barcode of FIG. 3(b) and ID29 to ID37 including the unstructured barcode);
iii) The RNA sequence of interest flanked on both sides by two bases of spacers (solid line in FIG. 3(b), and point mutations in the sequence are indicated by triangles.
iv) A 3'-cassette required for generating the RNA library via *in-vitro* transcription (IVT), the reverse transcription and for the library preparation for sequencing. (broken line on the 3'-side in FIG. 3(b)).

(Design of the second library)

The outline of the barcode sequence and library structure used for the second library is shown in FIG. 4. The RNA according to this design comprises two barcodes of in-library barcodes (the first barcode) and batch barcodes (the second barcode). The RNA can be divided into the following four parts from 5' to 3':

i) The same 5'-cassette as used in the first library design;
ii) The same barcode sequence as used in the first library design;
iii) The RNA sequence of interest flanked on both sides by two bases of spacers;
iv) A 12 nucleotide linker sequence to enhance primer binding.

Prior to performing *in vitro* transcription (IVT), the following two parts were further attached by polymerase chain reaction (PCR).

v) Four different batch barcodes. This barcode is the same sequence for all target RNAs in one batch.
vi) The same 3'-cassette as used in the first library design.

The nucleotide sequences of the primers used for amplification of the second library are as follows.

[Table 1]

| Name | Sequence | SEQ ID No. |
|---|---|---|
| Pr_d2a | 5'-GAACCGGACCGAAGCCCGATTTGGAGCGGAAGCCGCTCACCGT TGACCAGTTGTGCAC-3' | 2 |
| Pr_d2b | 5'-GAACCGGACCGAAGCCCGATTTGGGCCACAAGGTGGCCACCGT TGACCAGTTGTGCAC-3' | 3 |
| Pr_d2c | 5'-GAACCGGACCGAAGCCCGATTTGACCTCTGTTACAGAGGTACCG TTGACCAGTTGTGCAC-3' | 4 |
| Pr_d2d | 5'-GAACCGGACCGAAGCCCGATTTGACACACCTTAGGTGTGTACCG TTGACCAGTTGTGCAC-3' | 5 |

(Synthesis of DNA strand)

The libraries and primers described above were ordered to synthesize from Integrated DNA Technologies, Inc. (IDT) in DNA form. As controls, two individual RNA probes (ID1 and ID32, respectively) with structured or unstructured barcode sequences designed in the first library were synthesized.

(Synthesis of RNA from DNA)

First, the libraries were amplified by PCR with Platinum™ SuperFi™ PCR Master Mix (from Thermo Fisher Scientific). For the first library and two individual single-stranded RNAs in this library, the forward primer having a 5'-cassette sequence downstream of the T7 RNA polymerase promoter sequence (IVT recognition site: 5'-TAATACGACTCACTATAG-3' (SEQ ID NO:6)) and the reverse primer having a sequence complementary to the 3'-cassette sequence were used. As reverse primers for preparing the second library, four different batches were made using Pr_d2a (SEQ ID NO:2), Pr_d2b (SEQ ID NO:3), Pr_d2c (SEQ ID NO:4), and Pr_d2d (SEQ ID NO:5) to attach the barcodes. In all reactions, each primer was added to a final concentration of 500 nM, and the template was added to a total concentration of 0.4 nM. The reaction volume was 25 $\mu$L. All PCRs were performed on a ProFlex™ PCR system from Thermo Fisher Scientific.

After heating to 98°C for 30 seconds, 3-step PCR was performed at 98°C for 10 seconds, 68°C for 10 seconds, 72°C for 15 seconds. After the last cycle, the temperature was held at 72°C for 5 minutes followed by cooling to 4°C. 2.5 $\mu$L of Exonuclease I (New England Biolabs Inc.) was added to each tube and incubated at 37°C for 15 min before cooling to 4°C again. Purification is performed by the method described in Monarch® PCR & DNA Cleanup Kit (5 $\mu$g) (New England Biolabs Inc.). For the final elution, 10 $\mu$L of DNA elution buffer was used. A NanoDrop™ One (Thermo Fisher Scientific) was used to measure final concentrations.

The prepared double-stranded DNA is used as template for the IVT reaction using the MEGAshortscript™ T7 Transcription Kit (Thermo Fisher Scientific). The reaction was prepared according to the manual. The reaction volume was 20 $\mu$L and the template concentration was 100 nM. The reaction was incubated for 6 hours at 37°C followed by treatment with TURBO DNase (supplied with the kit) for 15 minutes at 37°C. The RNA was then purified with RNA Clean & Concentrator-25 from Zymo Research.

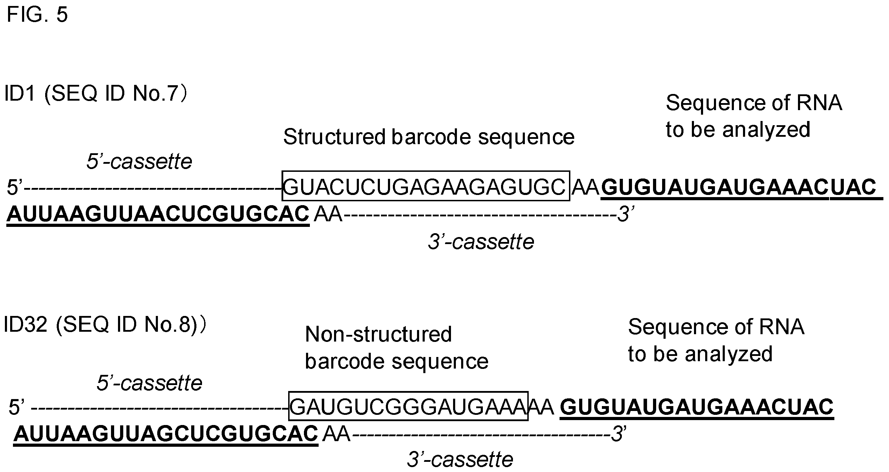

FIG. 5 shows nucleotide sequences of ID1 (SEQ ID NO:7) and ID32 (SEQ ID NO:8) synthesized as individual strands among RNA probes contained in the first library synthesized by *in vitro* transcription. In FIG. 5, each barcode sequence portion was surrounded by a square, and the target RNA sequence was underlined.

(Preparation of RNA for structural profiling)

Two different chemical modification reagents were used for RNA modification. Dimethyl sulfate (DMS) of the methylating agent purchased from Sigma Aldrich and SHAPE reagent 2-methylnicotinic acid imidazolide (NAI). For the experiments with both modification agents, the same preparation of RNA was used: 250 ng of RNA (single strand or pool) dissolved in 6 $\mu$L water were incubated at 95°C for 2 minutes and snap cooled on ice for at least 2 minutes. Then, 3 $\mu$L of 3.3x folding buffer were added. The sample incubated at 37°C for 20 minutes (1$\times$ folding buffer consists of 100 mM HEPES (pH 8.0), 100 mM NaCl, 10 mM MgCl$_2$).

(Structure profiling modification with NAI)

1 $\mu$L of 1000 mM NAI solution was added to empty 0.2 mL PCR tubes. Tubes were maintained on ice until just prior to addition of RNA. At 37° C, 9 $\mu$L of sample containing RNA was added to NAI. After pipetting and mixing, the samples were left at 37° C for 10 minutes.

After the reaction was stopped, the RNA was purified with an RNA Clean and Concentrator-5 kit from Zymo Research. The final elution volume was 15 $\mu$L. For each RNA sample modified with NAI, control samples treated in the same manner using 1 $\mu$L DMSO instead of NAI were prepared.

(Structure profiling modification with DMS)

1 $\mu$L of 50% DMS with ethanol was added to 9 $\mu$L of samples containing RNA previously prepared at 37° C. The samples were incubated at 37° C for 6 minutes. The reaction was stopped with mixing and incubation with 5 $\mu$L $\beta$-mercaptoethanol at 37°C for 2 minutes. Next, the RNA was purified by the RNA Clean and Concentrator-5 kit of Zymo Research. The final elution volume is 15 $\mu$L. For each RNA sample modified with DMS, control samples treated in the same manner using 1 $\mu$L of 50% aqueous ethanol instead of DMS were prepared.

(Reverse transcription for mutational profiling)

The modified RNA samples were subjected to a reverse transcription reaction using a reverse primer having a sequence complementary to the 3'-cassette sequence. In the case of the NAI-modified RNA, the enzyme Superscript™ II reverse transcriptase (Thermo Fisher Scientific) was used in the presence of manganese. For the DMS-modified RNA, TGIRT™-III enzyme (InGex) was used. In both cases, 1 $\mu$L of 2 $\mu$M reverse primer was mixed with 2 $\mu$L of 10 mM dNTP (New England Biolabs) and 7 $\mu$L of previously modified RNA. The samples were annealed in a ProFlex™ PCR system from Thermo Fisher Scientific (85°C 1 min→65°C 10 min→4°C hold) which was also used for the reverse transcription step. Next, 9 $\mu$L of 2.22x MaP-buffer was added and incubated at room temperature for 2 min. Then, 1 $\mu$L of reverse transcriptase was added, and the samples are placed in the cycler for reverse transcription (see Table 2).

[Table 2]

|  | DMS-treated RNA | NAI-treated RNA |
|---|---|---|
| 1xMapbuffer | 100 mM Tris (pH 8.0), 75 mM KCl, 3 mM MgCl$_2$, 1 M betaine, 10 mM DTT | 50 mM Tris (pH 8.0), 75 mM KCl, 6 mM MnCl$_2$, 1 M betaine, 10 mM DTT |
| Enzyme | TGIRT™-III | Superscript™ II Reverse Transcriptase |
| Temperature protocol | 25°C 5 min → 57°C 90 min →90°C 10 min → 4°C hold | 25°C 10 min → 42°C 90 min → [50°C 2 min → 42°C 2min] × 15 → 72°C 10 min → 4°C hold |

Upon completion of reverse transcription, 1 $\mu$L of RNaseH was added, and the samples were incubated at 37°C for 20 min. Purification was performed according to the protocol using AMPure XP (manufactured by Beckman Coulter). For elution, 14 $\mu$L of water was added to the dried beads, mixed thoroughly, and incubated at room temperature for 10 minutes. After elution, 12.5 $\mu$L of supernatant was collected.

(Preparation of next generation sequencing library)

Two PCRs, amplicon PCR and index PCR, were performed to prepare the library. For the amplicon PCR, 1 ng of the reverse transcription products were used in a reaction volume of 25 $\mu$L. The other reaction components were 1x Platinum™ SuperFi™ PCR Master Mix and 1× SuperFi GC Enhancer (both from Thermo Fisher Scientific), and 500 nM of forward primer and reverse primer. The samples were placed to a ProFlex™ PCR system. After initial heating to 98°C for 30 seconds, 3-step PCR was performed at 98°C for 10 seconds, 64°C for 10 seconds, and 72°C for 20 seconds. After the last cycle, the temperature was held at 72°C for 5 minutes followed by cooling to 4°C. For purification, the DNA CLEANUP AND CONCENTRATION protocol for the Monarch® PCR & DNA Cleanup Kit (5 $\mu$g) (New England Biolabs Inc.) was used. For the final elution, 8 $\mu$L of DNA elution buffer were used. The products are ready to attach indices for Illumina sequencing.

Next, index PCR is done with 1 ng of the amplicon PCR products in a reaction volume of 25 $\mu$L. The other reaction components are 1x Platinum™ SuperFi™ PCR Master Mix and 1 $\mu$M index primers from the Nextera XT Index Kit v2(Illumina). The samples were placed to ProFlex™ PCR system. After initial heating to 98°C for 30 seconds, 3 cycles of PCR were performed at 98°C for 10 seconds, 55°C for 10 seconds and 72°C for 20 seconds. After the last cycle, the temperature was held at 72°C for 5 minutes followed by cooling to 4°C. Purification was performed using AMPure XP (manufactured by Beckman Coulter). For elution, 13 $\mu$L of water was added to the dried beads, mixed thoroughly, and incubated at room temperature for 10 minutes. After elution, 12 $\mu$L of supernatant was collected. The samples are then pooled together for sequencing.

(Next Generation Sequencing)

For sequencing, a NextSeq 500/550 Mid Output Kit v2.5 (150 Cycles) using paired-end reads and standard read primers was used.

(Aligning and Data Analysis)

The adapters of the FASTQ files were first trimmed, and then the processed reads in the FASTQ files were aligned using the STAR aligner software. Mutations, deletions and insertions were counted for further analysis.

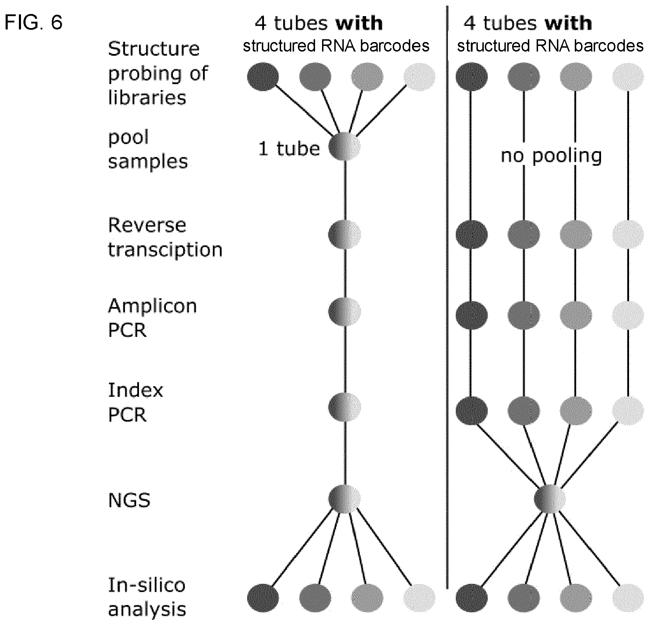

FIG. 6 is a schematic diagram showing a flow of mutational profiling performed using the second library. Four libraries, each chemically modified separately, were combined into one tube to perform a reverse transcription reaction.

On the other hand, four tubes were prepared as comparative control samples, which were obtained by individual reverse transcription reactions on the four libraries.

Results and Discussion

(Barcodes to distinguish sequences in RNA libraries)

A library with the first design was used to test if the barcodes help to distinguish similar sequences in mutational profiling experiments. The Levenshtein distance was used as an index to measure the similarity of the character string to measure the similarity of two sequences. This distance indicates the minimum number of insertions, deletions, and mutations to convert one sequence to another. Without the addition of barcodes, this number is 1 or 2 for any pair of sequences in the library. When a barcode is added, the Levenshtein distance is 7 or more. Thus, even with an increase in mutation rate expected in mutation profiling experiments, the sequence can be correctly identified. In addition to the complete library, two single sequences of the library (ID1 and ID32) were used as controls. ID1 contains structured barcodes, while ID32 contains unstructured barcodes (see FIG. 5).

The three RNA samples (the library and two single sequence controls) were either modified with NAI or DMS as described above. Including the control samples without NAI/DMS, the total sample size is 12. The samples, including a single sequence, were aligned to all sequences of the first library. Data were analyzed by counting the mutation (M), deletion (D) and insertion (I) of each nucleotide for each ID. The mutation rate (mut) was calculated by summing M, D, I and dividing by the total number of reads at a base position. In order to reduce noise due to sequence-specific mutation, the mutation rate of the unmodified sample was subtracted from the mutation rate of the NAI/DMS-modified sample to determine the delta mutation rate of the following formula (1).

```
Delta mutation rate = modified mutation rate - unmodified
mutation rate     (1)
```

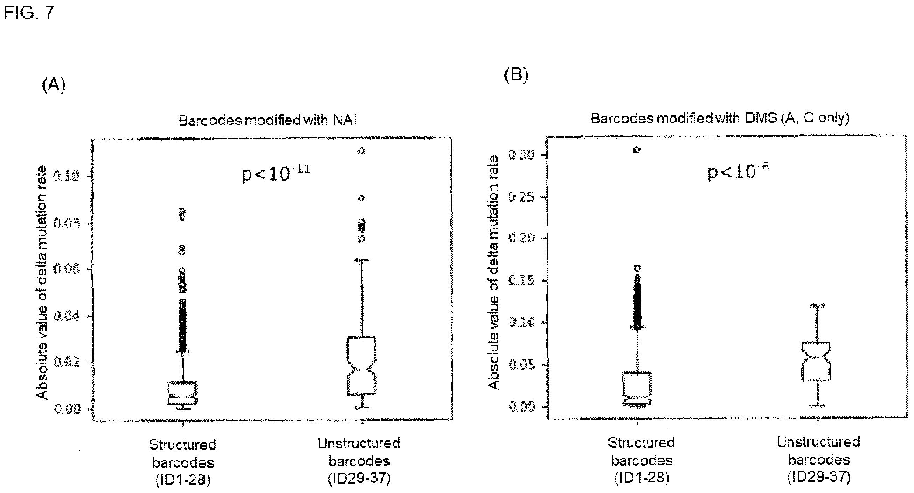

The results are shown in FIG. 7. FIG. 7(a) is a box plot showing the absolute delta mutation rate for all nucleotides in the barcode sequence of the first library modified with NAI. FIG. 7(b) shows the results of analyzing samples treated with DMS in the same manner. In FIG. 7, the box plot elements are defined as follows: centre line, median; box limits, upper and lower quartiles; points, outliers. These results indicate that the absolute value of the delta mutation rate of the structured barcode sequence (ID1 to ID28) is significantly lower than the absolute value of the delta mutation rate of the unstructured barcode sequence (ID29 to ID37) even when treated with any modifying agent of NAI or DMS. That is, it is shown that mutation can be prevented by structuring the barcode.

(Barcodes to distinguish different RNA libraries)

The second library was used to experiment if barcodes help to distinguish different versions of RNA libraries in a common pool of all versions. Thus, to prepare the second library, batch barcodes (second barcodes) was assigned to RNA, and the library was differentiated into 4 different versions using primers Pr_d2a, Pr_d2b, Pr_d2c, Pr_d2d prior to *in vitro* transcription. As shown in FIG. 6, four different versions of the RNA library were modified with NAI or DMS or treated as respective controls. After the purification step, pooled samples were prepared for each treatment condition by mixing equal amounts of four versions of the library. Each of the four different versions of the library and pooled samples were processed in the same manner in successive steps.

These delta mutation rates are shown in FIG. 8(a) and FIG. 8(b) in which the target sequence of ID1 is plotted as the x-axis. Delta mutation rates showed all four groups of the first and second libraries (data from pooled samples). In the case of NAI (FIG. 8(a)), delta mutation rates of the first library and the second library are slightly different, but the mutation rate is high in the unbound nucleotide region for all libraries, indicating that structural probing reflects information on secondary structure. The ViennaRNA package was used for RNA structure prediction. In the case of DMS (FIG. 8(b)), the difference between libraries is not so significant, but similar to NAI, the structural information shows a value with a higher delta mutation rate in a region predicted to be unbound (unconstrained). Under the conditions in which the experiment was performed, DMS has low modification efficiency of bases G and U, and thus only bases C and A show higher mutation rates. Comparing the pooled and individually processed sample results, good overlap is seen between the curves of the graphs (FIG. 8(c) and FIG. 8(d)). Therefore, pooling is considered not to have a significant effect on the results of the experiment, and structured RNA worked as a barcode.

(Secondary structure information for RNA libraries based on the second library design)

FIG. 8 shows only a single-ID mutation profile. The mutation profiles of all IDs were then analyzed and compared to the secondary structure predicted in the ViennaRNA package. FIG. 9 is a violin plot showing the absolute values of delta mutation rates of regions predicted to form base pairs (black regions in FIG. 9) and regions predicted to be unbound (grey regions in FIG. 9) separately when the second library is chemically modified with NAI or DMS, respectively, alone or in a pool. Samples of FIG. 9(a) are treated with NAI, and samples of FIG. 9(b) are treated with DMS. Among the IDs shown on the x-axis, IDs 1 to 28 include structured barcode sequences, and ID 29 to 37 include unstructured barcode sequences. This result also shows that the distribution of four individual samples (left side of "biolin" in FIG. 9) and pooled samples (right side of "biolin" in FIG. 9) are very similar. In the case of DMS, only the positions of bases A and C are considered.

This result indicates that the absolute value of the delta mutation rate of the unbound region is high when NAI (FIG. 9(a)) or DMS (FIG. 9(b)) is used for each ID sample, indicating that secondary structure information can be acquired for each sequence in the library. Furthermore, there was no significant difference in mean and distribution width (standard deviation) between the pooled and unpooled samples. It is shown that when modified by DMS, the S/N ratio is improved, and the overlap of the distribution of the modified RNA and the unmodified RNA is reduced. On the other hand, a remarkable overlap of distribution is often observed in the unstructured barcodes (IDs 29 to 37) as opposed to the structured barcodes (IDs 1 to 28). This means that the unstructured barcode does not match the data obtained by RNA structure prediction, indicating that the unstructured barcode affected the structure of the RNA to be analyzed.

[Example 2]

The accuracy of barcode identification by using structured barcodes

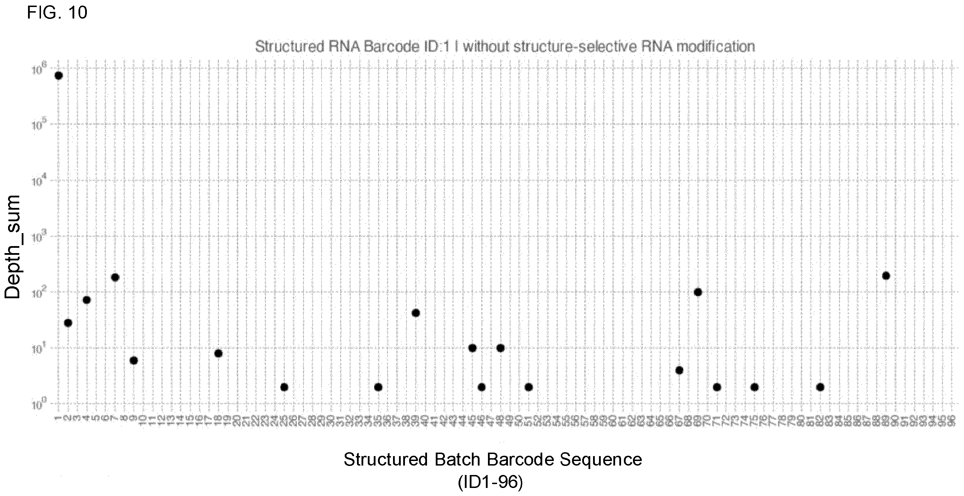

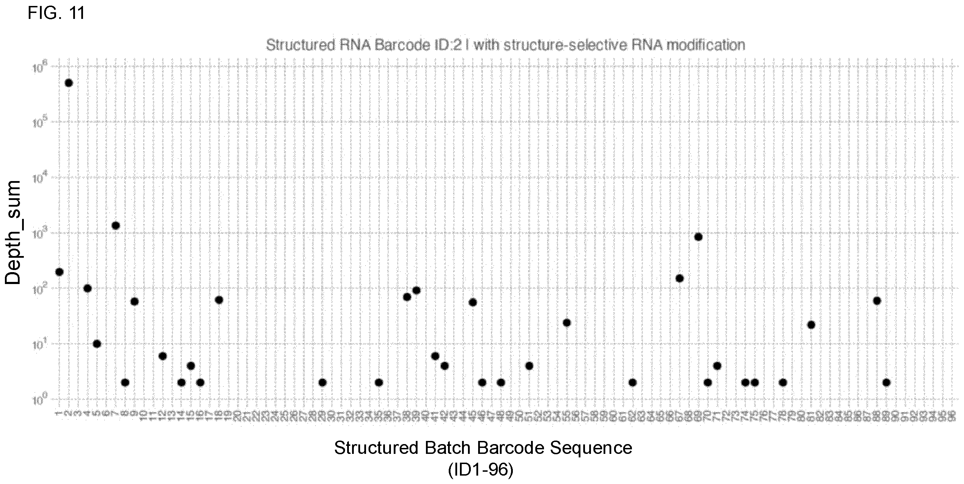

The 96 structured batch barcodes were prepared for a multiplexed library (RNA probe library) in which 54 kinds of RNA structures were mixed in total. Then, for mapping, these different batch barcodes were attached to all 54 RNA structures included in the library to create 96×54 reference files. An RNA probe library to which two kinds of batch barcodes having different IDs were added was synthesized *in vitro,* and mutation profiling experiments using DMS were performed. Corresponding indexes were assigned to different structured batch barcodes for validation experiments and next generation sequencing analysis was performed. All reads obtained were then mapped to a reference file. In this analysis, mapping was performed using STAR aligner software. The results are shown in FIG. 10 and FIG. 11.

FIG. 10 shows the result of an experiment using the structured batch barcode 1. The horizontal axis represents the ID determined by the sequence and mapping, and the vertical axis represents the total number of reads (Depth_sum). The mutation profile reaction using structured batch barcode 1 does not use a modifying agent, and does not have an effect of mutations selective to RNA structures. As a result, most of the structured batch barcode 1 has been correctly determined as ID1. However, since the number of reads of other IDs is very small, that is, 1/1000 to 1/10000 or less, for the correct ID 1, interpretation of data of the mutation profile is not affected.

FIG. 11 is an experiment using the structured batch barcode 2. The horizontal axis represents the ID determined by the sequence and mapping, and the vertical axis represents the total number of reads (Depth_sum). A modifying reagent is used in a mutation profile reaction system using structured batch barcode 2, and mutagenesis occurs in a higher order structure selective manner of RNA. In FIG. 11, the result shows an increased number of misclassified reads assigned to other than ID2, but the majority of the misclassified reads are assigned to ID2. This result indicates that the barcode functioned for sample assignment in the same way as the result shown in FIG. 10. In addition, the total number of reads of an ID (determined to be other than ID2) determined erroneously as compared with a correct ID (determined to be ID2) is very small, i.e., 1/100 to 10000 or less, and thus does not affect interpretation of data of a mutation profile.

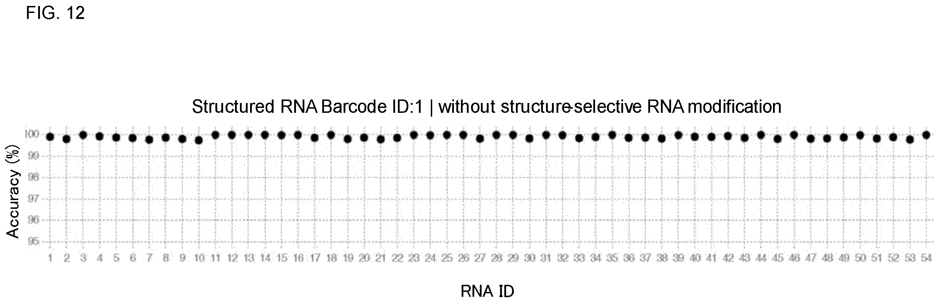

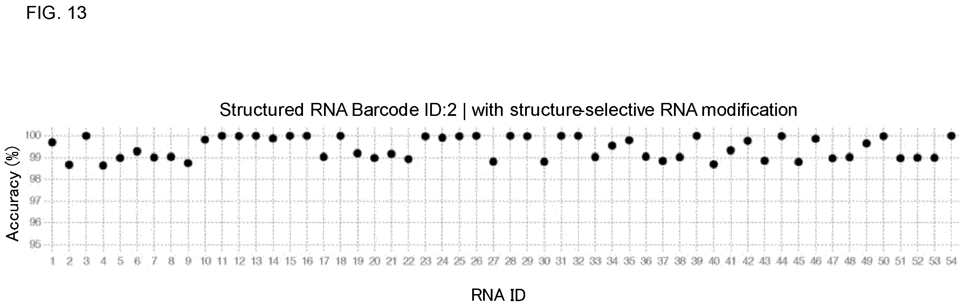

The accuracy (the ratio determined to be the correct ID) was confirmed for each of 54 types of RNAs in the library (FIG. 12 and FIG. 13). As a result, the accuracy in unmodified conditions is averaged 99.91%, and the accuracy in modified conditions is averaged 99.44%. High accuracy was maintained even under the mutation introduction conditions.

As described above, the structured batch barcode can clearly distinguish the correct barcode ID from other incorrect IDs without impairing the mapping accuracy in the mutation profile, and thus is useful for multiplexing in which a plurality of different conditions are mixed at the same time.

[Example 3]

Effect of multiplexing by combination of a barcode and another barcode (index)

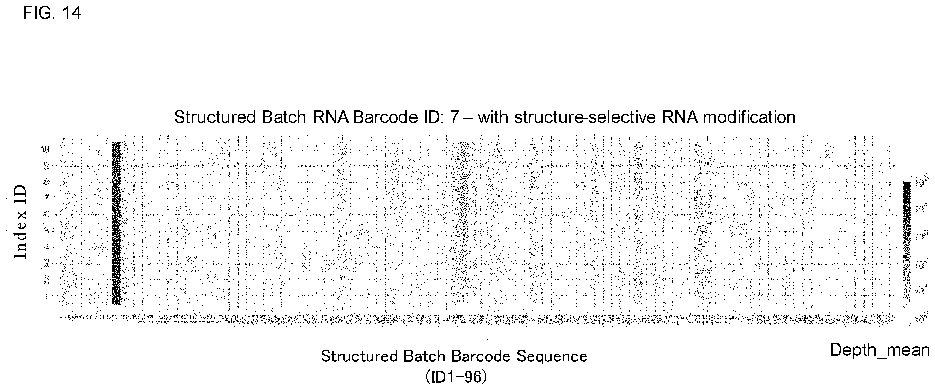

After completing the mutational profiling reaction using RNA and converting it to DNA, it can be combined with commercially available index primers (e.g., Nextera XT Index Kit <Illumina Inc. >) to increase the complexity of the sample origin and conditions. In FIG. 14, the vertical axis represents the ID determined by the illumina index primer (functioning as a barcode). The horizontal axis represents the ID determined by structured RNA barcode ID7 prepared in Example 2. The color scale indicates the average value of the number of reads.

From the above, it was found that the structured batch barcode (ID) can be identified with high accuracy in any index primer. That is, it can be said that the number of samples can be expanded to a large scale by combining a plurality of forms of DNA barcodes in addition to the batch barcode. For example, 960 conditions of $10 \times 96$ can be set by using 10 index primers and 96 structured barcodes.

[Example 4]

Orthogonality in next generation sequence analysis using structured barcodes

32 kinds of structured batch barcodes were prepared for a multiplexed library (RNA probe library) in which 1500 kinds of RNA probes different in the whole were mixed. For mapping, all 1500 RNAs were then given different batch barcodes and RNA probe libraries were synthesized *in vitro* with $32 \times 1500$ (48000) reference files. Next, profile analysis was performed using the RNA probe library group to which the structured batch barcode was attached. For validation experiments, 32 different structured batch barcodes were all indexed (Index ID) using 32 different illumina index primers, and then sequencing analysis was performed using a next generation sequencer (MiSeq <Illumina>). The file was then distributed into 32 files by index. If the barcode functions correctly, for example, the demultiplexed file corresponding to index ID 1 includes an RNA probe library to which structured batch barcode ID 1 is assigned. All reads obtained were then mapped to a reference file. In this analysis, mapping was performed using STAR aligner software.

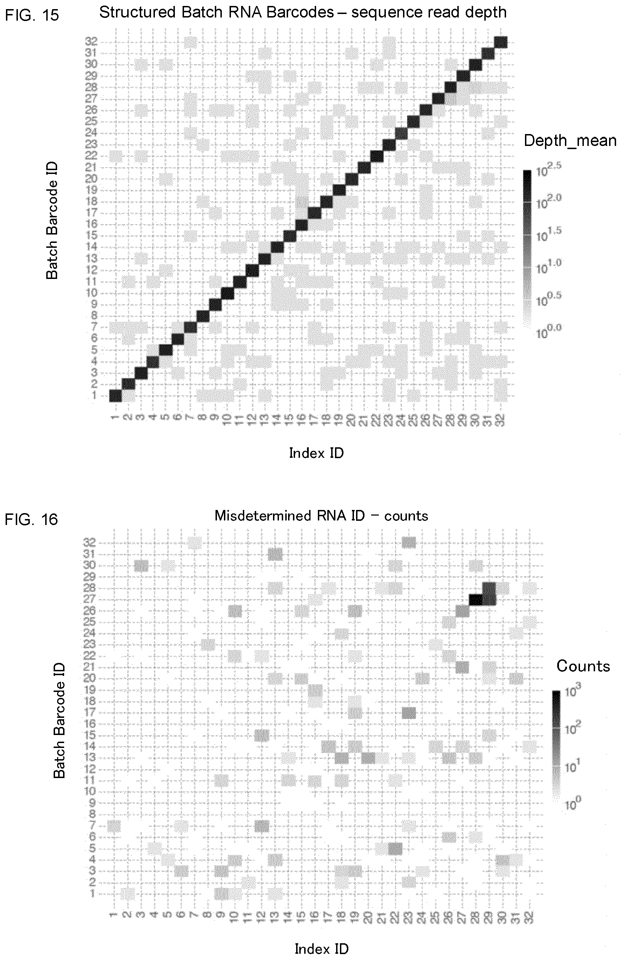

In FIG. 15, the horizontal axis represents a correct index (Index ID), and the vertical axis represents a structured batch barcode ID (Batch Barcode ID) determined by sequence and mapping. The color of the heat map indicates the average value (Depth_mean) of the number of mapped reads in the RNA probe library. As shown in FIG. 15, it was confirmed that all structured barcodes were allocated to the correct ID. Furthermore, as shown in FIG. 16, it was found that the erroneous determination occurs for almost 0 or less than 10 kinds of 1500 kinds of RNA in the library, and the influence on the RNA probes of the entire library is very small.

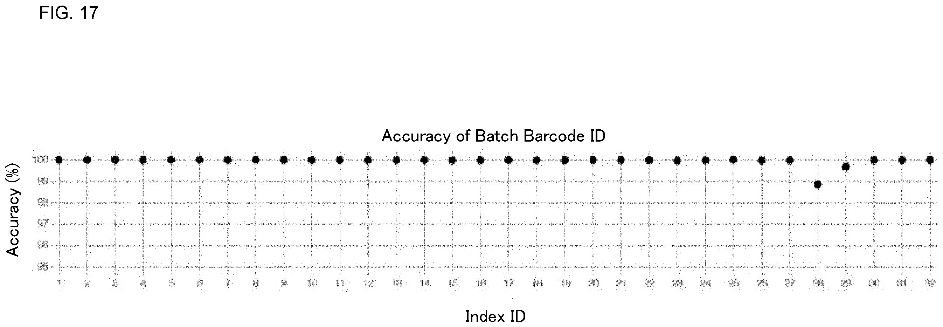

In addition, since the number of RNA read counts for these misdetermined RNA is approximately 1/100 to 10000 or less as compared with the correct ID, it can be said that the influence is further small and the misdetermination does not affect interpretation of the result of the profile (FIG. 17). For this reason, the structured batch barcode can be said to have high orthogonality as intended, indicating that it functions as a barcode. In FIG. 16, there are data points that contains misdetermined 800 kinds and 130 kinds of RNAs. However, since there is no similarity in barcode sequence and continuous occurrence between adjacent tubes, it is determined that these data points are caused by contamination due to artificial mistakes, and there is no problem due to a specific structured barcode.

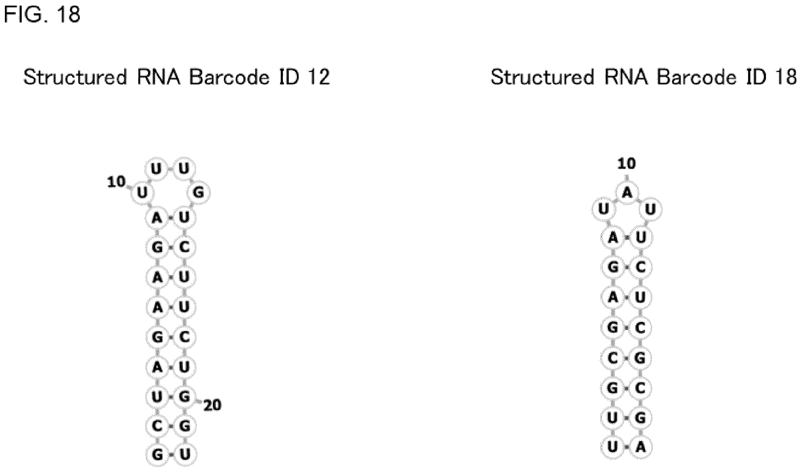

FIG. 18 shows examples (ID12 and ID28) of the structured barcode sequence used in the present example. The structured barcode RNA of ID12 comprises a loop structure of four bases with a 22 base length: 5'-GCUAGAAGAUUUGUCUUCUGGU-3' (SEQ ID NO:9). On the other hand, the structured barcode RNA of ID28 contains a loop structure of three bases in 19 bases in length: 5'-UUGCAGAUUCUCGCGA-3' (SEQ ID NO:10). In this way, the structured barcode can change not only the nucleotide sequence but also the length and the higher-order structure, and thus the combination thereof can be further expanded.

As described above, the structured barcode can multiplex the structural probing experiments under a plurality of reaction conditions. As an application, a multiplexed structural probing can be performed after a plurality of different reaction types and experimental conditions are prepared, and the influence of these different conditions on the RNA structure can be screened on a large scale. For example, the method exemplified in the following Reference Documents [1] to [3] can be used to extend to screening in which a plurality of types of molecules and conditions known to cause structural changes are evaluated at once.

References

[1] Komatsu, K. R., Taya, T., Matsumoto, S., Miyashita, E., Kashida, S., & Saito, H. (2020). RNA structure-wide discovery of functional interactions with multiplexed RNA motif library. Nature communications, 11(1), 1-14.

[2] Tapsin, S., Sun, M., Shen, Y., Zhang, H., Lim, X. N., Susanto, T. T.... & Wan. Y., (2018). Genome-wide identification of natural RNA aptamers in prokaryotes and eukaryotes. Nature communications, 9(1), 1-10.

[3] Corley, M., Flynn, R. A., Lee, B., Blue, S. M., Chang, H. Y., & Yeo, G. W. (2020), Footprinting SHAPE-eCLIP Reveals Transcriptome-wide Hydrogen Bonds at RNA-Protein Interfaces. Molecular Cell, 80(5), 903-914.

**Claims**

1. A method for analyzing a higher-order structure of RNA comprising the steps of:

   (a) preparing one or a plurality of RNA probes, each comprising an RNA to be analyzed attached to a barcode sequence,
   (b) contacting the RNA probes and an RNA modification reagent,
   (c) detecting a position and a frequency of modified nucleotides in a sequence of the RNA probe obtained in step (b) ,

   wherein each of the barcode sequences has a structure that has a reduced reactivity with the RNA modification reagent.

2. The method of claim 1 wherein step (c) comprises:

   (c1) synthesizing complementary DNAs using a mixture of the RNA probes obtained in step (b) as a template by a reverse transcriptase,
   (c2) determining nucleotide sequences of the complementary DNAs and aligning the nucleotide sequences comprising the barcode sequence, and
   (c3) detecting a position and a frequency of mutations occurred in the aligned nucleotide sequences.

3. The method of claim 1 or 2, wherein the barcode sequence does not form a base pair, in a case where the RNA modification reagent selectively modifies a bound nucleotide in the RNA probe.

4. The method of claim 1 or 2, wherein the barcode sequence forms a structure comprising a plurality of base pairs in a case where the RNA modification reagent selectively modifies an unbound nucleotide in the RNA probe.

5. The method of claim 4, wherein the structure comprising a plurality of base pairs is a complementary double-stranded structure, a triple chain structure or a quadruple chain structure.

6. The method of claim 4 or 5, wherein the plurality of base pairs are present in a stem region of a stem-loop structure or a pseudo-knot structure.

7. The method of any one of claims 4 to 6, wherein the structure comprising a plurality of base pairs is a stem-loop structure comprising one or more bulge and/or an internal loop in the stem region.

8. The method of any one of claims 4 to 7, wherein the structure comprising a plurality of pairs is deposited in PDB (Protein Data Bank) or a derivative thereof.

9. The method of any one of claims 1 to 8, wherein the RNA to be analyzed comprises at least one RNA structural motif.

10. An RNA probe comprising an RNA to be analyzed attached to a barcode sequence that forms a structure comprising a plurality of base pairs.

11. The RNA probe of claim 10, wherein the structure comprising a plurality of base pairs is a complementary double-stranded structure, a triple chain structure or a quadruple chain structure.

12. The RNA probe of claim 10 or 11, wherein the plurality of base pairs are present in a stem region of a stem-loop structure or a pseudo-knot structure.

13. An RNA probe library comprising a plurality of RNA probes, each of which comprises an RNA to be analyzed attached to a barcode sequence that forms a structure comprising a plurality of base pairs.

14. A group of RNA probe libraries comprising two or more replicates of the RNA probe libraries of claim 13, wherein all the replicated RNA probes further comprise second barcode sequences, and the second barcode sequences are identical within each of the RNA probe libraries, while distinguishable between other RNA probe libraries.

FIG. 1

S10

```
Preparation of RNA probe
```

S20

```
Modification of RNA probe
```

S30

```
Detection of modified bases
```

S40

```
Display of detection results
```

FIG. 2

S10

Preparation of RNA probe

S20

Modification of RNA probe

S30

S31

Synthesis of cDNA by reverse transcriptase

S32

Determination and alignment of nucleotide sequence

S33

Detection of modified bases

S40

Display of detection results

EP 4 202 056 A1

FIG. 3

(a)

```
    A  G
  G      A
  U  -  A
  C  -  G
  U  -  A
  C  -  G
  A  -  U
  U  ·  G
  G  -  C
  5'     3'
```

(b)

FIG. 4

## 1 DNA library with 37 barcoded sequences

## 4 RNA libraries with 4 different batch barcodes

batch
barcodes

FIG. 5

ID1 (SEQ ID No.7）

Structured barcode sequence

Sequence of RNA
to be analyzed

*5'-cassette*

5'-----------------------------------GUACUCUGAGAAGAGUGC AA**GUGUAUGAUGAAACUAC**

**AUUAAGUUAACUCGUGCAC** AA---------------------------------3'

*3'-cassette*

ID32 (SEQ ID No.8)）

Non-structured
barcode sequence

Sequence of RNA
to be analyzed

*5'-cassette*

5' ----------------------------------GAUGUCGGGAUGAAA AA **GUGUAUGAUGAAACUAC**

**AUUAAGUUAGCUCGUGCAC** AA---------------------------------3'

*3'-cassette*

FIG. 6

FIG. 7

FIG. 8

FIG. 9

FIG. 10

Structured RNA Barcode ID:1 I without structure-selective RNA modification

Depth_sum

Structured Batch Barcode Sequence
(ID1-96)

EP 4 202 056 A1

FIG. 11

EP 4 202 056 A1

Structured RNA Barcode ID:2 I with structure-selective RNA modification

Depth_sum

Structured Batch Barcode Sequence
(ID1-96)

FIG. 12

Structured RNA Barcode ID:1 | without structure-selective RNA modification

FIG. 13

Structured RNA Barcode ID:2 | with structure-selective RNA modification

FIG. 14

Structured Batch RNA Barcode ID: 7 – with structure-selective RNA modification

Structured Batch Barcode Sequence (ID1–96)

Index ID

Depth_mean

FIG. 15    Structured Batch RNA Barcodes – sequence read depth

FIG. 16    Misdetermined RNA ID – counts

FIG. 17

Accuracy of Batch Barcode ID

FIG. 18

Structured RNA Barcode ID 12          Structured RNA Barcode ID 18

## INTERNATIONAL SEARCH REPORT

| International application No. |
|---|
| **PCT/JP2021/042250** |

**A.    CLASSIFICATION OF SUBJECT MATTER**

*C12Q 1/6806*(2018.01)i; *C12N 15/09*(2006.01)i; *C40B 40/06*(2006.01)i
FI:    C12Q1/6806 Z; C12N15/09 Z; C40B40/06

According to International Patent Classification (IPC) or to both national classification and IPC

**B.    FIELDS SEARCHED**

Minimum documentation searched (classification system followed by classification symbols)

C12Q1/6806; C12N15/09; C40B40/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | WO 2018/003809 A1 (UNIV KYOTO) 04 January 2018 (2018-01-04) claims, examples, fig. 2 | 10-14 |
| A | JP 6612220 B2 (THE UNIVERSITY OF NORTH CAROLINA AT CHAPEL HILL) 27 November 2019 (2019-11-27) entire text | 1-14 |
| A | ZUBRADT, M. et al. DMS-MaPseq for genome-wide or targeted RNA structure probing in vivo. Nat. Methods. 2017, vol. 14, no. 1, pp. 75-82 entire text | 1-14 |
| A | KWOK, C. K. et al. The RNA structurome: transcriptome-wide structure probing with next-generation sequencing. Trends Biochem. Sci. 2015, vol. 40, no. 4, pp. 221-232 entire text | 1-14 |
| A | STROBEL, E. J. et al. RNA systems biology: uniting functional discoveries and structural tools to understand global roles of RNAs. Curr. Opin. Biotechnol. 2016, vol. 39, pp. 182-191 entire text | 1-14 |

☐ Further documents are listed in the continuation of Box C.        ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **14 January 2022** | **01 February 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2021/042250** |

**Box No. I**     **Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

a.  ☑  forming part of the international application as filed:

☑  in the form of an Annex C/ST.25 text file.

☐  on paper or in the form of an image file.

b.  ☐  furnished together with the international application under PCT Rule 13*ter*.1(a) for the purposes of international search only in the form of an Annex C/ST.25 text file.

c.  ☐  furnished subsequent to the international filing date for the purposes of international search only:

☐  in the form of an Annex C/ST.25 text file (Rule 13*ter*.1(a)).

☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/JP2021/042250**

| Patent document cited in search report | | | Publication date (day/month/year) | Patent family member(s) | | | Publication date (day/month/year) |
|---|---|---|---|---|---|---|---|
| WO | 2018/003809 | A1 | 04 January 2018 | (Family: none) | | | |
| JP | 6612220 | B2 | 27 November 2019 | US | 2016/0244818 | A1 | |
| | | | | WO | 2015/054247 | A1 | |
| | | | | EP | 3055413 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2020191550 A
- JP 6612220 B

- WO 2018003809 A

### Non-patent literature cited in the description

- **MEGAN ZUBRADT et al.** DMS-Mapseq for genome-wide or targeted RNA structure probing in vivo. *Nat Methods,* 2017, vol. 14, 75-82
- **AW, J.G.A. ; LIM, S.W. ; WANG, J.X. et al.** Determination of isoform-specific RNA structure with nanopore long reads. *Nat Biotechnol,* 2020, https://doi.org/10.1038/s41587-020-0712-z
- **HAMADA, M. et al.** *Bioinformatics,* 2009, vol. 25, 465-473
- **SATO, K. et al.** *Methods Biochem. Anal.,* 2011, vol. 27, i85-i93
- **GRILLO, G. et al.** *Nucl. Acids Res.,* 2010, vol. 38, D75-D80
- **MOKREJS, M. et al.** *Nucl. Acids Res.,* 2010, vol. 38, D131-D136
- **BENSON, D. et al.** *Nucl. Acids Res.,* 2013, vol. 41, D36-D42
- **RNACENTRAL CONSORTIUM.** *Nucl. Acids Res.,* 2015, vol. 43, D123-D129
- **NAWROCKI, E. P. et al.** *Nucl. Acids Res.,* 2015, vol. 43, D130-D137
- **BERKOWITZ, N. D. et al.** *BMC Bioinformatics,* 2016, vol. 17, 215

- **GARALDE, D. R. et al.** Highly parallel direct RNA sequencing on an array of nanopores. *Nat. Methods,* 2018
- **WORKMAN, R.E. et al.** Nanopore native RNA sequencing of a human poly(A) transcriptome. *Nat. Methods,* 2019, vol. 16, 1297-1305
- **WILLIAM STEPHENSON et al.** Direct detection of RNA modifications and structure using single molecule nanopore. *BioRxiv,* 01 June 2020
- **KOMATSU, K. R. ; TAYA, T. ; MATSUMOTO, S. ; MIYASHITA, E. ; KASHIDA, S. ; SAITO, H.** RNA structure-wide discovery of functional interactions with multiplexed RNA motif library. *Nature communications,* 2020, vol. 11 (1), 1-14
- **TAPSIN, S. ; SUN, M. ; SHEN, Y. ; ZHANG, H. ; LIM, X. N. ; SUSANTO, T. T. ; WAN. Y.** Genome-wide identification of natural RNA aptamers in prokaryotes and eukaryotes. *Nature communications,* 2018, vol. 9 (1), 1-10
- **CORLEY, M. ; FLYNN, R. A. ; LEE, B. ; BLUE, S. M. ; CHANG, H. Y. ; YEO, G. W.** Footprinting SHAPE-eCLIP Reveals Transcriptome-wide Hydrogen Bonds at RNA-Protein Interfaces. *Molecular Cell,* 2020, vol. 80 (5), 903-914